# EUROPÄISCHE PATENTSCHRIFT

(11) **EP 2 207 876 B1**
(45) Veröffentlichungstag und Bekanntmachung des Hinweises auf die Patenterteilung: **26.08.2015**
(21) Anmeldenummer: 08847246.9
(22) Anmeldetag: 07.11.2008
(51) Int. Cl.: C12N 9/02, C07D 239/00, C07D 405/00

(54) **STEREOSPEZIFISCHE HYDROXYLIERUNG**
STEREO-SPECIFIC HYDROXYLATION
HYDROXYLATION STÉRÉOSPÉCIFIQUE

(30) Priorität: 07.11.2007 DE 102007052900
(43) Veröffentlichungstag der Anmeldung: 21.07.2010
(73) Patentinhaber: Rheinische Friedrich-Wilhelms-Universität Bonn, 53113 Bonn (DE); Bitop AG, 58453 Witten (DE)
(72) Erfinder: GALINSKI, Erwin, A., 48308 Senden (DE); STEIN, Marlene, 53125 Bonn (DE); URES, Andrea, 54636 Röhl (DE); SCHWARZ, Thomas, 68199 Mannheim (DE)
(74) Vertreter: Thiel, Christian
(86) Internationale Anmeldenummer: PCT/EP2008/009414
(87) Internationale Veröffentlichungsnummer: WO 2009/059783

(56) Entgegenhaltungen:
- WO-A-01/38500
- GRAMMEL N: "Molekulargenetische und biochemische Analyse der Biosynthese von 2-Methyl-4-carboxy-3,4,5,6-tetrahydropyrim idin und seinem 5-Hydroxyderivat, zwei salzstressinduzierbaren Osmolyten, in Streptomyces chrysomallus" 1999, DISSERTATION, TECHNISCHE UNIVERSITÄT BERLIN , XP002511939 Gefunden im Internet: URL:http://edocs.tu-berlin.de/diss/2000/gr ammel_nicolas.pdf> das ganze Dokument, insbesondere S. 64, 65 und 79-83
- VOSS P: "Synthese von kompatiblen Soluten mit ectoinanaloger Struktur und Charakterisierung des protektiven Effektes auf biochemische Modellsysteme und Echerichia coli" 2002, DISSERTATION, WESTFÄLISCHE WILHELMS-UNIVERSITÄT MÜNSTER , XP002511940 Gefunden im Internet: URL:http://deposit.ddb.de/cgi-bin/dokserv? idn=967452503&dok_var=d1&dok_ext=pdf&filen ame=967452503.pdf>
- PRABHU JULIA ET AL: "Functional expression of the ectoine hydroxylase gene (thpD) from Streptomyces chrysomallus in Halomonas elongata." APPLIED AND ENVIRONMENTAL MICROBIOLOGY MAY 2004, Bd. 70, Nr. 5, Mai 2004 (2004-05), Seiten 3130-3132, XP002511938 ISSN: 0099-2240
- SAUER T ET AL: "BACTERIAL MILKING: A NOVEL BIOPROCESS FOR PRODUCTION OF COMPATIBLE SOLUTES" BIOTECHNOLOGY AND BIOENGINEERING, WILEY & SONS, HOBOKEN, NJ, US, Bd. 57, Nr. 3, 1. Januar 1998 (1998-01-01), Seiten 306-313, XP000919323 ISSN: 0006-3592

## Beschreibung

Die Erfindung betrifft ein Verfahren zur stereospezifischen Hydroxylierung von cyclischen α-Amino-S-carbonsäurederivaten zu cyclischen α-Amino-β-hydroxy-S-carbonsäureverbindungen, wobei die α-Aminfunktion in das Ringsystem eingebunden ist und die Hydroxyl- und Säuregruppe trans konfiguriert sind, unter Verwendung von Mikroorganismen mit Hydroxylaseaktivität in einem Produktionsmedium, bei dem die S-Carbonsäurederivate dem Produktionsmedium zugesetzt werden und in Gegenwart von Sauerstoff und eines Co-Substrats unter Nutzung osmotisch aktivierter Transportsysteme des Mikroorganismus im Mikroorganismus hydroxyliert und anschließend aktiv oder passiv aus dem Mikroorganismus freigesetzt werden, um aus dem Produktionsmedium gewonnen zu werden.

Stoffumwandlungen in nur wenigen katalytischen Schritten und unter Beteiligung von biologischen Systemen (Enzymen) werden auch Biotransformationen genannt und der "weißen" Biotechnologie zugerechnet, die insbesondere unter dem Aspekt der Nachhaltigkeit zunehmend zu einer wichtigen Stütze der chemischen Industrie wird. Grundsätzlich sind dabei katalytische Systeme bevorzugt, die ein möglichst breites Spektrum an Substraten umsetzen. Im Falle von Oxidations-/Reduktions-Reaktionen, wie z. B. Hydroxylierungsreaktionen, stellt die Regeneration der dazu benötigten Co-Faktoren ein technisches Problem dar. Sofern die Regeneration des Co-Faktors nur in Ganzzellsystemen - also in intakten lebenden oder ruhenden Mikroorganismen - erfolgen kann, sind damit eine Reihe von Schwierigkeiten verbunden. Dies ist zum Einen der in der Regel geschwindigkeitslimitierende Transport des Substrats in die Zellen hinein, zum Zweiten die Verwendung eines preiswerten Substrats für die Regeneration des Co-Substräts und zum Dritten der Export des Produkts aus den Zellen in das Medium, aus dem das Produkt dann gewonnen werden kann.

In stereospezifischen Hydroxylierungen unter Beteiligung der Co-Faktoren NADH bzw. NADPH wird das Problem der Co-Faktor-Regenerierung dadurch gelöst, dass ein zweites enzymatisches System, zum Beispiel die Formiat-Dehydrogenase, für die Regeneration des Co-Faktors in dem Produzentenstamm oder einen so genannten Membranreaktor eingebracht wird. Das dazu benötigte Substrat, in dem genannten Beispiel Formiat, wird bei der Reaktion verbraucht. 2-Oxoglutarat abhängige Dioxygenasen für Hydroxylierungsreaktionen unter Beteiligung von molekularem Sauerstoff sind zwar bekannt, bisher aber nicht zur praktischen Anwendung gelangt, weil das Co-Substrat zu teuer ist, um in der Reaktion verbraucht zu werden und Ganzzellsysteme sich bislang als ebenfalls unwirtschaftlich erwiesen haben.

Aus N. Grammel, "Molekulargenetische und biochemische Analyse der Biosynthese von 2-Methyl-4-Carboxy-3,4,5,6-Tetrahydropyrimidin und seinem 5-Hydroxyderivat, zwei Salzstress induzierbaren Osmolyten, in Streptomyces chrysomallus" (1999), Dissertation, Technische Universität Berlin, und der WO2001/038500A2 ist ein Enzym mit der Tetrahydropyrimidin-Dioxygenase-Aktivität von *Streptomyces chrysomallus* bekannt, das in vitro zur Hydroxylierung von Ectoin zu Hydroxyectoin in Gegenwart von 2-Oxoglutarat eingesetzt wurde. Die Expression der für das Enzym kodierenden Gensequenz in *Streptomyces lividans* und *E.coli* wird angesprochen. Zur Expression des Enzyms in der lebenden Zelle wird auf die Notwendigkeit der vorherigen Permeabilisierung der Zelle hingewiesen, um das Inkontaktkommen des Enzyms mit dem zu hydroxylierenden Tetrahydropyrimidin zu begünstigen. Auf die Problematik der Freisetzung des hydroxylierten Produkts aus der lebenden Zelle wird nicht eingegangen.

Ectoin ist ein Tetrahydropyrimidin, das in zahlreichen Bakterien gebildet wird, beispielsweise in *Halomonas elongata* durch die Ectoinbiosynthesegene. Die ebenfalls dort vorhandene Ectoin-Hydroxylase kann Ectoin in Hydroxyectoin überführen. Allerdings wird immer nur ein Teil des gebildeten Ectoins auch in Hydroxyectoin umgewandelt, in der Regel deutlich weniger als 50 %. Da die Trennung von Ectoin und Hydroxyectoin aufwendig ist, ist die Gewinnung von reinem Hydroxyectoin zeitraubend und kostspielig.

Eine Reihe potentieller Ectoin-Hydroxylasen sind in folgenden Mikroorganismen identifiziert worden:

| **Stamm** | **Protein** | **Sequenz identität [%]** |
|---|---|---|
| *Halomonas elongata* DSM 2581^{T} | Ectoin-Hydroxylase (EctD) | 100 |
| *Chromohalobacter salexigens* DSM 3043 ^{T} | Phytanoyl-CoA-Dioxygenase | 75 |
| *Bordetella parapertussis* | putative L-Prolin-4-hydroxylase | 51 |
| *Bordetella bronchiseptica* RB50 | putative L-Prolin-4-hydroxylase | 51 |
| *Streptomyces avermitilis* MA-4680 | putative Ectoin-Hydroxylase | 50 |
| *Nocardia farcinica* IFM 10152 | putative Ectoin-Hydroxylase | 50 |
| *Streptomyces coelicolor* A3(2) | putative Ectoin-Hydroxylase | 49 |
| *Streptomyces chrysmallus* | Ectoin-Hydroxylase (ThpD) | 48 |
| *Bacillus clausii* KSM-K16 | L-Prolin-4-hydroxylase | 46 |
| *Dactylosporangium sp.* | L-Prolin-4-hydroxylase | 33 |

Die in-vitro Biokatalyse von Ectoin zu Hydroxyectoin wie auch von Ectoinanaloga zu den hydroxylierten Verbindungen mit der Hydroxylase von C. *salexigens* wurde nachgewiesen. Erste Ergebnisse deuten auf eine entsprechende Aktivität der anderen Hydroxylasen hin.

Der Erfindung liegt die Aufgabe zugrunde, ein biologisches System bereitzustellen, das die stereospezifische Hydroxylierung insbesondere von Ectoin, aber auch von ähnlich gebauten Molekülen mit Hilfe einer von 2-Oxoglutarat abhängigen Dioxygenase in einem Ganzzellsystem in wirtschaftlich bedeutsamen Mengen ermöglicht. Von besonderem Interesse als Zielverbindungen sind solche aus der Substanzklasse der kompatiblen Solute, die in chemischen, kosmetischen und pharmazeutischen Herstellungsprozessen bzw. Produkten eingesetzt werden können. Ein solches Ganzzellsystem soll über die notwendigen Transportsysteme zur Einschleusung der Edukte aus dem Kulturmedium verfügen und in der Lage sein, die Produkte über Transportsysteme oder auf anderem Wege, beispielsweise über sogenannte Leckagen, auszuschleusen.

Diese Aufgabe wird mit einem Verfahren gelöst, bei dem als Mirkroorganismen solche der Gattungen *Escherichia, Bacillus, Corynebacterium, Klebsiella, Marinococcus* oder *Halomonas* eingesetz werden.

Bei dem Verfahren werden zur stereospezifischen Hydroxylierung von α-Amino-S-carbonsäurederivaten - im folgenden S-Carbonsäurederivate - zu α-Amino-β-hydroxy-S-carbonsäureverbindungen unter Verwendung von Mikroorganismen mit Hydroxylaseaktivität in einem Produktionsmedium die S-Carbonsäurederivate dem Produktionsmedium zugesetzt und in Gegenwart von Sauerstoff und eines Co-Substrates unter Nutzung von etwa osmotisch aktivierten Transportsystemen des Mikroorganismus im Mikroorganismus hydroxyliert und anschließend aus dem Mikroorganismus aktiv oder passiv freigesetzt, um aus dem Produktionsmedium gewonnen zu werden. Die Hydroxylierung erfolgt stereotaktisch dergestalt, dass die Hydroxylcarbonsäuregruppe in cyclischen Verbindungen trans konfiguriert sind.

Bevorzugte Ausführungsformen sind Gegenstand der Unteransprüche.

Unter aktiver Freisetzung wird die Freisetzung über ein Transportsystem verstanden, unter passiver Freisetzung über mechanosensitive Kanäle oder Leckagen.

Derivate und Verbindungen im Sinne der Erfindung sind insbesondere die Säuren selbst, aber auch ihre Salze, Ester und Amide.

Das erfindungsgemäße Verfahren ist insbesondere geeignet, das Edukt vollständig und stereoselektiv zum hydroxylierten Produkt umzusetzen, welches dann aus dem Produktionsmedium nach standardisierten Verfahren gewonnen werden kann. Es versteht sich, dass das hydroxylierte Produkt auch aus dem Mikroorganismus gewonnen werden kann.

Bei dem Mikroorganismus kann es sich um einen solchen handeln, der die Hydroxylaseaktivität von Natur aus aufweist. In diesem Fall ist sicherzustellen, dass die notwendigen Transportsysteme für die Ein- und Ausschleusung von Edukt und Produkt vorhanden sind und das Hydroxylasegen dauerhaft exprimiert wird. Im Falle von extremophilen Bakterien der Gattung *Halomonas, Marinococcus* u.a., die die Hydroxylaseaktivität aufweisen, sind zwar die entsprechenden Mechanismen zur Überwindung der Membranbarriere vorhanden, das Hydroxylasegen wird aber nicht dauerhaft exprimiert. Die dauerhafte Expression des natürlich vorhandenen Hydroxylasegens kann z. B. dadurch erreicht werden, dass das Gen durch rekombinative Verfahren genomisch unter die Kontrolle eines anderen natürlicherweise vorhandenen Promotors gestellt wird, oder ein geeigneter Promotor auf an und für sich bekannte Art und Weise in den Mikroorganismus eingeschleust wird, um dort den natürlicherweise vorhandenen Promotor zu ersetzen. Ein geeigneter Promotor ist beispielsweise der osmotisch induzierte Promotor PromA aus *Marinococcus halophilus* bzw. die osmotisch induzierbaren Promotoren des Ectoinbiosynthesegenclusters aus *Halomonas elongata* (sowie Modifikationen derselben) und andere Stressinduzierte Promotoren oder Stationärphasenpromotoren. Alternativ kann die dauerhafte Expression des natürlicherweise vorhandenen Hydroxylasegens auch erreicht werden, indem das Gen auf einem natürlicherweise vorhandenen Plasmid oder auf einem eingeschleusten Vektor unter die Kontrolle geeigneter Promotoren gestellt wird. Es versteht sich, dass auch durch Mutation rekombinant veränderte (natürliche) Promotoren in Frage kommen.

Vorzugsweise handelt es sich aber um einen mit einem Hydroxylasegen transformierten Mikroorganismus, der auf übliche Art und Weise dazu gebracht werden kann, die Hydroxylase zu exprimieren, beispielsweise durch den Einsatz eines Induktors. Geeignete Induktoren sind Laktose oder IPTG. Bevorzugt ist aber die Induktion der Hydroxylaseaktivität durch Einstellen einer Stresssituation in dem Kulturmedium, beispielsweise durch Temperaturveränderung oder osmotische Veränderung, oder aber durch den Eintritt einer Mangelsituation wie z. B. beim Übergang in die stationäre Phase.

Erfindungsgemäße Mikroorganismen sind solche der Gattungen *Escherichia, Klebsiella, Halomonas, Bacillus, Corynebacterium und Marinococcus, insbesondere Escherichia coli, Halomonas elongata, Bacillus subtilis, Marinococcus halophilus und Corynebacterium glutamicum.* Bevorzugt wird die Hydroxylase aus *H. elongata* DSM 2581 eingesetzt, die auf an und für sich bekannte Weise in den Zielorganismus transferiert wird, beispielsweise über ein Plasmid. Geeignete Plasmide sind z. B. solche vom pET-Typ (z. B. pET22b(+) der Fa. NOVAGEN, Madison, USA), vom pBBR-Typ (Kovach ME, Elzer PH, Hill SD, Robertson GT, Farris MA, Roop MR, Peterson KM (1995) Gene 166:175-176) und pHSG575 und Derivate (Takeshita S, Sato M, Toba M, Masahashi W, Hashimoto-Gotoh T (1987) Gene 61:63-74). Das Hydroxylasegen (ectD) aus *Halomonas elongata* DSM 2581 ist beschrieben, siehe A. Ures et al., Ectoine Hydroxylase (ectD) from Halomonas elongata. Posterbeitrag zur Jahrestagung der VAAM in Göttingen vom 25.09. bis 28.09.2005.

Als geeignet für die Durchführung des erfindungsgemäßen Verfahrens hat sich u.a. der Mikroorganismus *E.coli* BL21 (STRATAGENE, La Jolla, USA) erwiesen. Das Verfahren ist entsprechend in anderen erfindungsgemäßen Mikroorganismen anwendbar, die die Edukte über Transportsysteme aufnehmen und die Produkte in das Medium abgeben, etwa andere Proteobakterien oder Gram-positive Bakterien der Gattung *Bacillus* oder *Corynebacterium.*

Bei allen hier angegebenen Mikroorganismen und Plasmiden handelt es sich um solche, die käuflich erwerblich, über Hinterlegungsstellen frei erhältlich sind bzw. ausführlich in der Literatur beschrieben sind.

Als Co-Substrat für die vorstehend genannte Dioxygenase aus *Halomonas elongata* dient naturgemäß 2-Oxoglutarat. Als Kohlenstoffquelle können übliche eingesetzt werden; insbesondere besteht auch die Möglichkeit, Glycerin als außerordentlich preiswerte Kohlenstoffquelle einzusetzen. Die Regeneration erfolgt auf bekannte Weise über Acetyl-CoA und den Citratcyclus.

Von besonderer Bedeutung sind die Transportsysteme der zum Einsatz kommenden Mikroorganismen, die geeignet sein müssen, Edukte durch die Zellmembran zu transportieren. Bei *E.coli* handelt es sich beispielsweise um die gut charakterisierten Transporter ProP und ProU, die beide ein sehr breites Substratspektrum haben und lineare wie zyklische Moleküle durch die Membranbarriere transportieren können. So werden beispielsweise ohne Weiteres Glycinbetain, Dimethylglycin, Glycin, Homobetain, Prolin, Prolinbetain, 3,4-Dihydroprolin, Pipecolinsäure, Taurin, Carnitin, γ-Butyrobetain, Trigonellin und Ectoin durch die Zellmembran geschleust, siehe G. Gousbet et al., Pepicolic acid is an osmoprotectant for Escherichia coli taken up by the general osmoporters ProU and ProP, Microbiology 140 (1994), 2415-2422; B. Kempf et al., Uptake and synthesis of compatible solutes as microbial stress responses to high-osmolality environments, Arch. Microbiol. 170 (1998), 319-330. Die Effizienz des Verfahrens kann dadurch beeinflusst werden, dass vorhandene Transportsysteme ganz oder teilweise deletiert, mutagenisiert oder auf andere Art und Weise in ihrer Selektivität beeinflusst werden.

Das erfindungsgemäße Verfahren kann auf übliche Art und Weise in einem Nähr- oder Produktionsmedium durchgeführt werden, wobei sowohl Batch-Verfahren als auch Fed-Batch- und kontinuierliche Verfahren zum Einsatz kommen können. Eine kontinuierliche Vorgehensweise ist bevorzugt. Die hydroxylierten Produkte können auf übliche Art und Weise mit Hilfe chromatographischer Verfahren isoliert werden, beispielsweise durch Säulenchromatographie.

Bei den S-Carbonsäurederivaten, die als Ausgangsverbindungen dienen, handelt es sich um cyclische α-Amino-S-carbonsäurederivate, die die Grundstruktur von Aminosäuren haben und entsprechend zur Ausbildung von Zwitterionen in der Lage sind.

Besonders bevorzugt sind cyklische S-Carbonsäurederivate, bei denen eine α-Aminogruppe im Ringsystem angeordnet ist. Das Ringsystem kann 3 bis 8-gliedrig ausgebildet sein, wobei neben dem Stickstoffatom in der α-Position zur Carbonsäure ein oder mehrere weitere Stickstoff-, Sauerstoff- oder Schwefelatome vorhanden sein können. Der Ring kann weiter durch Alkylgruppen mit bis zu sechs Kohlenstoffatomen, Halogenatome, Amino-. Imino-, Nitro-, CN-, Hydroxy-, Ether- oder Estergruppen substituiert sein, wobei für die Ether- und Esterreste insbesondere Alkylreste mit bis zu sechs Kohlenstoffatomen in Frage kommen. Entsprechendes gilt naturgemäß auch für nicht-zyklische Edukte.

Es hat sich herausgestellt, dass das erfindungsgemäße Verfahren die Ausgangsverbindungen hochstereospezifisch in die trans-β-Hydroxy-S-carbonsäureverbindungen überführen kann. Die Umwandlung erfolgt nicht, wie im Stand der Technik, enzymatisch in einer Pufferlösung außerhalb der Zelle, sondern in der Zelle des transformierten Mikroorganismus selbst, der das Produkt dann wieder in das Medium abgibt.

Im Gegensatz zu anderen von 2-Oxoglutarat abhängigen Dioxygenasen ist die aus *Halomonas elongata,* die vorzugsweise eingesetzt wird, relativ wenig spezifisch und in der Lage, eine Reihe von Ausgangsverbindungen zu hydroxylieren. Das breite Substratspektrum wird ergänzt durch die Fähigkeit des Systems, das Edukt über Transportsysteme zur im Cytoplasma exprimierten Hydroxylase zu transportieren und das Produkt nach der Umsetzung ins Medium abzugeben. Dadurch wird die Zellmembran als Permeabilitätsbarriere überwunden und der Co-Faktor durch die Zellen selbst regeneriert. Das Produkt kann ohne Zerstörung der exprimierenden Zellen in nahezu beliebigen Mengen in sehr hohen Konzentrationen im Medium akkumuliert und daraus gewonnen werden.

Bei den für den Transport in die Zellen benutzten Membranproteinen (Transportern), handelt es sich im Falle von *E.coli,* wie angesprochen, vorzugsweise um die Osmolyttransporter proP und proU. Bei diesen handelt es sich um unspezifische Systeme, die neben den bevorzugten Substraten Glycinbetain und Prolinbetain u. a. auch Prolin, Ectoin und eine Reihe von Ectoinderivaten akzeptieren. Grundsätzlich sind alle Transportsysteme geeignet, die die Membranbarriere für die Edukte überwinden, also zum Beispiel auch andere Transportsysteme für Aminosäuren.

Die Transportsysteme ProP und ProU aus *E.coli* haben, wie schon dargestellt, ein breites Substratsystem. Bekannte Transportsysteme von *Bacillus subtilis* sind OpuA, OpuB, OpuC und OpuD, bei *Corynebacterium glutamicum* EctP, BetP, ProP und LcoP. Alle diese Transportsysteme akzeptieren ebenfalls ein breites Substratspektrum.

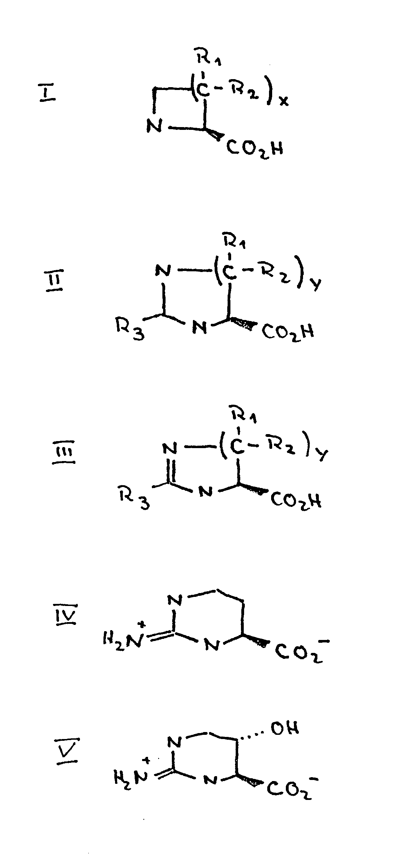

Das erfindungsgemäße Verfahren ermöglicht in einer wirtschaftlichen Weise die nahezu vollständige Umsetzung eines Substrats in ein β-Hydroxy-funktionalisiertes Produkt, das sich im Medium selbst anreichert. Es führt bei α-Aminocarbonsäurederivaten immer zu einer Stereokonfiguration mit der Hydroxylgruppe in trans-Stellung zur Carbonsäuregruppe. Eine Reihe von Produkten sind in Abbildung 1 angegeben. Von den genannten Substanzen sind Hydroxyectoin und ähnliche Derivate von besonderem Interesse, weil diese insbesondere als neuartige zellschützende Substanzen Verwendung in der Kosmetik und in Medizinprodukten finden können. Auf den hydroxylierten Monomeren aufbauende neuartige Biopolymere könnten beispielsweise als Amphotere, so genannte "Styling Polymere" in der Kosmetik oder zur Beschichtung von Oberflächen eingesetzt werden, um deren Biokompatibilität zu erhöhen. Nach dem Stand der Technik ist eine wirtschaftliche Herstellbarkeit dieser Stoffe für die genannten Anwendungen nicht möglich.

### Beispiel: Hydroxylierung von Ectoin und dgl.

Der Produktionsstamm E. coli BL21 enthält das Hydroxylasegen aus *Halomonas elongata* auf dem Plasmid pET22b(+)-EctD. Er wird in einem Medium der folgenden Zusammensetzung bei 37° C angezüchtet: 13,61 g/l KH₂PO₄, 4,21g/l KOH, 1,98 g/l (NH₄)SO₄, 0,25 g/l MgSO₄ x 7 H₂O, 1,1 mg/l FeSO₄ x 7 H₂O, 10 g/l NaCl 5 g/l Glucose, pH 7,0 eingestellt mit KOH, Carbenicillin 100 mg/l.

Das Medium wurde mit Sauerstoff belüftet, wobei die Sauerstoffsättigung durch Änderung des Druckluftzustroms (0,1 bis 0,5 l/min) und der Rührerdrehzahl (200 bis 600 upm) bei über 50% gehalten wurde. Bei Erreichen einer optischen Dichte von 0,6 (bei 600 nm) wurde durch Injektion von 1 ml IPTG-Lösung (23,8 mg/ml, steril filtriert) pro Liter die Expression induziert. Etwa eine Stunde nach Induktion erfolgte die sterile Zugabe des Substrats (Endkonzentration 2 bis 20 mM) ggf. in Kombination mit einer Kohlenstoffquelle zur Regeneration des Co-Faktors. Die Hydroxylierung des Substrats wurde durch Probennahme und anschließende HPLC-Analyse sowohl in den Zellen als auch im Medium verfolgt. Durch nachträgliche Zugabe von Substrat in Kombination mit einer C-Quelle (z. B. Glycerin) konnte der Ertrag gesteigert und die Konzentration des Produkts im Medium erhöht werden. Nach vollständigem Substratumsatz wurde die Kultur durch Zentrifugation von Kulturmedium abgetrennt und zur Isolierung des Produkts aufbereitet. Die Umsatzrate betrug mehr als 0,1 mmol/g Trockenbiomasse und Stunde.

Edukt und C-Quelle müssen in adäquaten Mengen gleichzeitig hinzugefügt werden. Im Falle der Verwendung von Glycerin als C-Quelle ist das molare Verhältnis 1:1.

Das Verfahren wurde beispielhaft mit Standardkonzentrationen von 2 bis 20 mM durchgeführt. Höhere Konzentrationen sind möglich. Der Grad der Sauerstoffsättigung ist nicht entscheidend; wichtig ist dass den Mikroorganismen genügend Sauerstoff zur Verfügung steht.

Das Verfahren wurde beispielhaft an dem Plasmid pET22b (+)-EctD durchgeführt. Plasmide vom pBBR-Typ und Low-Copy-Plasmide, beispielsweise pHSG 575, sind ebenfalls gut geeignet, insbesondere wenn das Hydroxylasegen aus *Halomonas elongata* unter der Kontrolle eines osmotisch induzierten Promotors, wie z. B. PromA aus *Marinococcus halophilus,* stress-induzierter Promotoren, Stationärphasenpromotoren oder anderer starker Promotoren steht.

Bevorzugte Edukte sind Verbindungen der Formeln I, II und III, bei denen R₁ und R₂ für Wasserstoff stehen und R₃ Wasserstoff, eine Alkylgruppe mit bis zu 6 Kohlenstoffatomen oder eine Aminogruppe ist. x steht für eine ganze Zahl von 1 bis 5, y für 1 bis 4.

Die Formel IV steht für Guanidiniumectoin mit einer S-Konfiguration an C₂. Die Verbindung entspricht der Formel III mit R₁=R₂=H, y=2 und R₃=NH2.

Auf der Produktseite steht R₂ für eine 3-Hydroxygruppe, die Trans zur S-Carboxylgruppe steht. Die Hydroxylierung erfolgt ausschließlich in der Trans-3-Position. Formel V gibt das Hydroxylierungsprodukt von Guanidiniumectoin wieder.

Es versteht sich, dass die Strukturen I bis V durch weitere Alkylgruppen mit bis zu 6 Kohlenstoffatomen, Halogenatome, Amino-, Imino-, Nitro-, CN-, Hydroyxy-, Ether- oder Estergruppen substituiert sein können.

Die Erfindung betrifft schließlich auch die neuen Verbindungen 3-Hydroxyhomoectoin, 3-Hydroxy-DHMICA, 3-Hydroxy-ADPC, 3-Hydroyxyguanidiniumectoin und 3-Hydroxy-2-azetidin-2-carbonsäure und deren Derivate, insbesondere Ester, Amide und Salze. Alle diese Verbindungen zeigen die Hydroxygruppe in der trans-Stellung zur Carbonsäure- bzw. Sulfonsäurefunktion. Sie betrifft ferner die aus den hydroxylierten Verbindungen durch alkalische Hydrolyse erhältlichen linearen Verbindungen: N-α-Acetyl- bzw. N-δ-Acetyl-2,5-diamino-3-hydroxy-valeriansäure, N-α-Acetyl- bzw. N-β-Acetyl-2,3-diamino-3-hydroxypropionsäure, 3-Hydroxyglutamin sowie deren Derivate, insbesondere Ester, Amide und Salze.

## Patentansprüche

1. Verfahren zur stereospezifischen Hydroxylierung von cyclischen α-Amino-S-carbonsäurederivaten zu cyclischen α-Amino-β-hydroxy-S-carbonsäureverbindungen, wobei die α-Aminfunktion in das Ringsystem eingebunden ist und die Hydroxyl- und Säuregruppe trans konfiguriert sind, unter Verwendung von Mikroorganismen mit Ectoin-Hydroxylaseaktivität in einem Produktionsmedium, bei dem die S-Carbonsäurederivate dem Produktionsmedium zugesetzt werden und in Gegenwart von Sauerstoff und eines Co-Substrats unter Nutzung osmotisch aktivierter Transportsysteme des Mikroorganismus im Mikroorganismus hydroxyliert und anschließend aktiv oder passiv aus dem Mikroorganismus freigesetzt werden, um aus dem Produktionsmedium gewonnen zu werden, **dadurch gekennzeichnet, dass** als Mikroorganismen solche der Gattungen Escherichia, Bacillus, Corynebacterium, Klebsiella, Marinococcus oder Halomonas eingesetzt werden.

2. Verfahren nach Anspruch 1, **dadurch gekennzeichnet, dass** der Mikroorganismus natürlicherweise ein Hydroxylasegen aufweist oder ein mit einem Hydroxylasegen transformierter Mikroorganismus ist.

3. Verfahren nach Anspruch 1 oder 2, **dadurch gekennzeichnet, dass** der Mikroorganismus ein mit einem Transportergen transformierter Mikroorganismus ist oder natürlicherweise ein geeignetes Transportersystem besitzt.

4. Verfahren nach einem der Ansprüche 1 bis 3, **dadurch gekennzeichnet, dass** die Gene unter der Kontrolle natürlicherweise vorhandener Promotoren dauerhaft zur Expression gebracht werden oder der Mikroorganismus ein mit einem natürlichen, mutierten oder gentechnisch veränderten Promotor transformierter Mikroorganismus ist.

5. Verfahren nach einem der vorstehenden Ansprüche, **dadurch gekennzeichnet, dass** das Hydroxylasegen aus Halomonas elongata stammt.

6. Verfahren nach Anspruch 5, **dadurch gekennzeichnet, dass** das Hydroxylasegen in einem Plasmid vom pET-Typ, vom pBBR-Typ oder pHSG 575 sowie Derivaten davon enthalten ist.

7. Verfahren nach einem der vorstehenden Ansprüche, **dadurch gekennzeichnet, dass** E.coli BL 21 als Mikroorganismus verwandt wird.

8. Verfahren nach einem der vorstehenden Ansprüche, **dadurch gekennzeichnet, dass** das Co-Substrat 2-Oxoglutarat mit Glykose, Acetat oder Glycerin als C-Quelle regeneriert wird.

9. Kontinuierliches Verfahren nach einem der vorstehenden Ansprüche.

10. Verfahren nach einem der vorstehenden Ansprüche, **gekennzeichnet durch** die chromatographische Gewinnung der trans-β-Hydroxycarbonsäureverbindungen.

11. Verfahren nach einem der vorstehenden Ansprüche, **dadurch gekennzeichnet, dass** ein α-Amino-S-carbonsäurederivat verwandt wird.

12. Verfahren nach einem der vorstehenden Ansprüche, **dadurch gekennzeichnet, dass** das S-Carbonsäurederivat ein kompatibles Solut ist.

13. Verfahren nach einem der vorstehenden Ansprüche, **dadurch gekennzeichnet, dass** das S-Carbonsäurederivat Ectoin, Homoectoin, DHMICA, ADPC, Prolin, Azetidin-2-carbonsäure oder Guanidiniumectoin ist.

14. Verfahren nach einem der vorstehenden Ansprüche, **gekennzeichnet durch** die vollständige Hydroxylierung von Ectoin zu Hydroxyectoin.

15. 3-Hydroxyhomoectoin, 3-Hydroxy-DHMICA, 3-Hydroxy-ADPC, 3-Hydroxyguanidiumectoin und 3-Hydroxy-2-azetidincarbonsäure, deren Ester und Salze sowie daraus durch alkalische Hydrolase erhältliche lineare Verbindungen.

## Claims

1. Method for the stereospecific hydroxylation of cyclic α-amino-S-carboxylic acid derivatives to form cyclic α-amino-β-hydroxy-S-carboxylic acid-compounds, wherein the α-amine function is integrated into the ring system and the hydroxyl and acid groups being trans-configured, using microorganism with ectoin hydroxylase activity in a production medium to which the S-carboxylic-acid derivatives are added and hydroxylated in the microorganism in the presence of oxygen and a cosubstrate using osmotically activated transporting systems of the microorganism and subsequently released actively or passively from the microorganism so as to be obtained from the production medium **characterized in that** the microorganism which stems from genus Escherichia, Bacillus Corynebacterium, Klebsiella, Marinococcus or Halomonas are used.

2. Method according to claim 1, **characterized in that** the microorganism naturally has a hydroxylase gene or is a microorganism transformed with a hydroxylase gene.

3. Method according to claim 1 or 2, **characterized in that** the microorganism is a microorganism transformed with a transporter gene or naturally possesses a suitable transporter system.

4. Method according to any one of the claims 1 to 3, **characterized in that** the genes under the control of naturally existing promoters are brought to permanent expression or the microorganism is a microorganism transformed by means of a natural, mutated or genetically modified promoter.

5. Method according to any one of the above claims, **characterized in that** the hydroxylase gene stems from Halomonas elongata.

6. Method according to claim 5, **characterized in that** the hydroxylase gene is contained in a plasmid of pET type, pBBR type or pHSG 575 as well as derivatives thereof.

7. Method according to any one of the above claims, **characterized in that** E.coli BL 21 is used as microorganism.

8. Method according to any one of the above claims, **characterized in that** the cosubstrate 2-oxoglutarate is regenerated with glycose, acetat or glycerine as C-source.

9. Continuous method according to any one of the above claims.

10. Method according to any one of the above claims, **characterized by** the chromatographic winning of the trans-β-hydroxy carboxylic-acid compounds.

11. Method according to any one of the above claims, **characterized in that** an α-amino-S-carboxylic acid derivative is used.

12. Method according to any one of the above claims, **characterized in that** the S-carboxylic acid derivative is a compatible solute.

13. Method according to any one of the above claims, **characterized in that** the S-carboxylic acid derivative is ectoine, homoectoine, DHMICA, ADPC, proline, acetidine-2-carboxylic acid or guanidinium ectoine.

14. Method according to any one of the above claims, **characterized by** the complete hydroxylation from ectoine to hydroxyectoine.

15. 3-hydroxyhomoectoine, 3-hydroxy-DHMICA, 3-hydroxy-ADPC, 3-hydroxyguanidium ectoine and 3-hydroxy-2-acetidine carboxylic acid, their esters and salts as well as linear compounds obtainable therefrom through alkaline hydrolysis.

## Revendications

1. Procédé pour l'hydroxylation stéréospécifique de dérivés cycliques d'acides α-amino-S-carboxyliques en des composés cycliques d'acides α-amino-β-hydroxy-S-carboxyliques, la fonction α-amine étant incorporée dans le système cyclique et le groupe hydroxyle et le groupe acide étant en configuration trans, par utilisation de microorganismes ayant une activité d'ectoïne-hydroxylase dans un milieu de production, procédé dans lequel les dérivés de l'acide S-carboxylique sont ajoutés au milieu de production et, en présence d'oxygène et d'un co-substrat, sont hydroxylés par utilisation de systèmes de transport à activation osmotique du microorganisme dans le microorganisme, puis sont libérés par voie active ou passive du microorganisme, pour être récupérés du milieu de production, **caractérisé en ce qu'**on utilise en tant que microorganismes des microorganismes des genres Escherichia, Bacillus, Corynebacterium, Klebsiella, Marinococcus ou Halomonas.

2. Procédé selon la revendication 1, **caractérisé en ce que** le microorganisme présente naturellement un gène de l'hydroxylase, ou est un microorganisme transformé par un gène de l'hydroxylase.

3. Procédé selon la revendication 1 ou 2, **caractérisé en ce que** le microorganisme est un microorganisme transformé par un gène transporteur, ou possède naturellement un système transporteur approprié.

4. Procédé selon l'une des revendications 1 à 3, **caractérisé en ce que** les gènes sont portés en permanence à expression sous le contrôle de promoteurs naturellement présents, ou **en ce que** le microorganisme est un microorganisme transformé avec un promoteur naturel, muté ou génétiquement modifié.

5. Procédé selon l'une des revendications précédentes, **caractérisé en ce que** le gène de l'hydroxylase provient de Halomonas elongata.

6. Procédé selon la revendication 5, **caractérisé en ce que** le gène de l'hydroxylase est contenu dans un plasmide du type pET, du type pBBR ou pHSG 575, ainsi que les dérivés de ceux-ci.

7. Procédé selon l'une des revendications précédentes, **caractérisé en ce qu'**on utilise en tant que microorganisme E. coli BL21.

8. Procédé selon l'une des revendications précédentes, **caractérisé en ce que** le co-substrat 2-oxoglutarate est régénéré avec du glucose, de l'acétate ou du glycérol en tant que source de C.

9. Procédé continu selon l'une des revendications précédentes.

10. Procédé selon l'une des revendications précédentes, **caractérisé par** l'obtention par chromatographie des composés de l'acide trans-β-hydroxycarboxylique.

11. Composé selon l'une des revendications précédentes, **caractérisé en ce qu'**on utilise un dérivé d'un acide α-amino-S-carboxylique.

12. Procédé selon l'une des revendications précédentes, **caractérisé en ce que** le dérivé de l'acide S-carboxylique est un soluté compatible.

13. Procédé selon l'une des revendications précédentes, **caractérisé en ce que** le dérivé de l'acide S-carboxylique est l'ectoïne, l'homoectoïne, le DHMICA, l'ADPC, la proline, l'acide azétidine-2-carboxylique ou la guanidinium-ectoïne.

14. Procédé selon l'une des revendications précédentes, **caractérisé par** l'hydroxylation complète de l'ectoïne en l'hydroxy-ectoïne.

15. 3-Hydroxyhomoectoïne, 3-hydroxy-DHMICA, 3-hydroxy-ADPC, 3-hydroxyguanidinium-ectoïne et acide 3-hydroxy-2-azétidine carboxylique, esters et sels de ceux-ci, ainsi que composés linéaires pouvant être obtenus à partir d'eux par une hydrolase alcaline.
